# EUROPÄISCHE PATENTSCHRIFT

(11) **EP 2 344 510 B1**
(45) Veröffentlichungstag und Bekanntmachung des Hinweises auf die Patenterteilung: **19.06.2013**
(21) Anmeldenummer: 09778691.7
(22) Anmeldetag: 24.09.2009
(51) Int. Cl.: C07F 9/6584, C07B 43/06

(54) **1,4,2-DIAZAPHOSPHOLIDIN-DERIVATE**
1,4,2-DIAZAPHOSPHOLIDINE DERIVATIVES
DÉRIVÉS DE LA 1,4,2-DIAZAPHOSPHOLIDINE

(30) Priorität: 04.10.2008 DE 102008050414
(43) Veröffentlichungstag der Anmeldung: 20.07.2011
(73) Patentinhaber: Bayer Intellectual Property GmbH, 40789 Monheim (DE)
(72) Erfinder: RICHTER, Frank, 51373 Leverkusen (DE)
(74) Vertreter: BIP Patents
(86) Internationale Anmeldenummer: PCT/EP2009/006893
(87) Internationale Veröffentlichungsnummer: WO 2010/037499

(56) Entgegenhaltungen:
- US-A- 3 551 527
- RICHTER, FRANK U.: "1,4,2-Diazaphospholidine-3,5-diones and Related Compounds: A Lecture on Unpredictability in Catalysis" CHEMISTRY--A EUROPEAN JOURNAL , 15(21), 5200-5202, S5200/1-S5200/13 CODEN: CEUJED; ISSN: 0947-6539, 3. April 2009 (2009-04-03), XP002561767

## Beschreibung

Die vorliegende Erfindung betrifft neuartige 1,4,2-Diazaphospholidin-Derivate, ein Verfahren zu deren Herstellung sowie die Verwendung als Katalysatoren.

Diazaphospholidine, insbes. Spezies mit P-N-C-N-C-Sequenz im Fünfring, sind selten und fallen in der Regel in mäßigen Ausbeuten bei Reaktionen an, die aufwändig zu synthetisierender Edukte bedürfen und oftmals mit der Bildung von Nebenprodukten einher gehen, die entweder schwer abtrennbar oder nur sehr aufwändig wieder in den Herstellkreiskauf des/der Edukte zurück zu führen sind (vgl. z.B. Zhurnal Obshchei. Khimii (1980) 50 (7), 1446-1451; Izv. Akad. Nauk, Ser. Khim. (1996) 7, 1857-1859; US 3551527, US 3980618, US 3989727, US 3980618, US 3965127, US 3904654).

Diazaphospholidine mit geminalen Carbonylgruppen und zwei P-N-Bindungen im Ring sind aus Phosphorus and Sulfur (1980) 8, 27 - 36 bekannt.

1,4,2-Diazaphospholidin-3,5-dione, 1,4,2-Diazaphospholidin-3,5-dithione sowie die entsprechenden Phosphacyclen mit R2-N= Substituent(en) an Stelle eines oder beider Chalkogenatome sind gänzlich unbekannt.

2,5-Dihydro-1H-phosphole sind oftmals mit den unsymmetrisch substituierten Pendants, den 2,3-Dihydro-1H-phospholen verunreinigt. Letztere sind als Katalysatoren für die Isocyanat-Oligomerisierung von Interesse, so dass diese vor ihrem Einsatz in die Oligomerisierungsreaktion zunächst von unerwünschten Bestandteilen wie den 2,5-Dihydro-isomeren befreit werden müssen.

Es wurde nun sehr überraschend gefunden, dass die Umsetzung der 2,5-Dihydro-1H-phosphole (3-Phospholene, symmetrische Phospholene) gemäß Formel (2) mit Isocyanaten R2-NCO (für X = O); Isothiocyanaten R2-NCS (für X = S) bzw. Carbodiimiden R2-NCN-R2 (für X = NR2) nahezu quantitativ in Bezug auf das 3-Phospholen zu 1,4,2-Diazaphospholidin-Strukturen der Formel (1) führt.

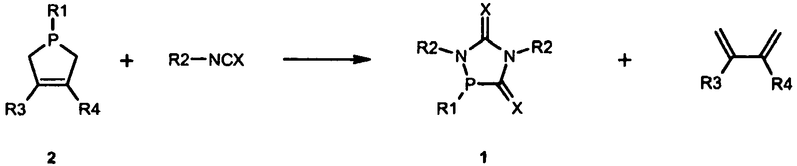

Diese Vorgehensweise eignet sich beispielsweise dazu, 2,5-Dihydro-1H-phosphole durch Derivatisierung von den für die NCO-Oligomerisation interessanten 2,3-Isomeren abzutrennen. Ferner sind Verbindungen der Formel (1) für Katalyseanwendungen von Interesse.

Gegenstand der vorliegenden Erfindung sind Verbindungen der Formel (1) sowie ein Verfahren zu deren Herstellung sowie die Verwendung als Katalysatoren.
- R1, R2: sind unabhängig voneinander gleiche oder verschiedene, ggf. durch Heteroatome (N, O, S, Halogen) substituierte bzw. unterbrochene, ggf. ein- oder mehrfach ungesättigte C₁-C₂₀-Alkyl-, -Alkenyl-, -Alkinyl- bzw. C₅-C₄₀-Arylgruppen.
- X: steht für gleiche oder verschiedene Substituenten aus der Gruppe O, S bzw. N-R2, wobei X bevorzugt Sauerstoff oder Schwefel ist.
- R3, R4: sind unabhängig voneinander gleiche oder verschiedene Substituenten aus der Gruppe H, Alkyl, Aryl, bevorzugt H und/oder Methyl.

Beim erfindungsgemäßen Verfahren zur Herstellung der vorstehend beschriebenen 1,4,2-Diazaphospholidin-Derivate werden 3-Phospholene der Formel (2) im molaren Verhältnis von 1 : 2 bis 1: 100, bevorzugt 1 : 2 bis 1 : 5, mit Isocyanaten R2-NCO, Isothiocyanaten R2-NCS oder Carbodiimiden R2-N=C=N-R2 umgesetzt.

Als Ausgangsstoffe für die erfindungsgemäße Umsetzung eigenen sich prinzipiell alle linear- oder verzweigt-aliphatischen, cycloaliphatischen und aromatischen Mono-, Di-, Tri- und Polyisocyanate, -isothiocyanate (-senföle) sowie -carbodiimide. Letztere können vorteilhafter Weise in einem Schritt aus den zu Grunde liegenden Isocyanaten generiert werden.

Beispielhaft seinen genannt: Methyliso(thio)cyanat, Ethyliso(thio)cyanat, Allyiso(thio)cyanat alle Regio- und Stereoisomeren der nachstehend genannten Mono-, Di-, Tri- sowie Polyiso-(thio)cyanate: Propyliso(thio)cyanate, Butyliso(thio)cyanate, Hexyliso(thio)cyanate, Octyliso(thio)cyanate, Alkoxyalkyliso(thio)cyanate wie z.B. Methoxypropyliso(thio)cyanat, Cyclohexyliso(thio)cyanat, (Methyl)cyclohexandiiso(thio)cyanate, Ethylcyclohexandiiso(thio)cyanate, Propylcyclohexandiiso(thio)cyanate, Methyl-diethyl-cyclohexandiiso(thio)cyanate, Phenyliso(thio)cyanat, Phenylendiiso(thio)cyanate, Tolyliso(thio)cyanate, Toluylendiiso(thio)cyanate, Bis(iso(thio)cyanatophenyl)methan und Polyphenyl-polymethylen-polyiso(thio)cyanate, wie sie beispielsweise durch Anilin-Formaldehyd-Kondensation und anschließende Phosgenierung hergestellt werden (MDI), Propandiiso(thio)cyanate, Butandiiso(thio)cyanate, Pentandiiso(thio)cyanate, Hexandiiso(thio)cyanate (z.B. Hexamethylendiisocyanat, HDI), Heptandiiso(thio)cyanate, Octandiiso(thio)cyanate, Nonandi- und triiso(thio)cyanate, Dekandi- und triiso(thio)cyanate, Undekandiund triiso(thio)cyanate, Dodecandi- und triiso(thio)cyanate, Isophorondiisocyanat (IPDI), Bis(iso(thio)cyanatocyclohexyl)methan (z.B. H_{12M}DI, Desmodur^{®}W), Iso(thio)cyanatomethyl-methylcyclohexane (z.B. 4(3)-Iso(thio)cyanatomethylcyclohexyliso(thio)cyanat) selbst sowie die aus ihnen zugänglichen Carbodiimide.

Hierbei ist es im Falle der Isocyanate belanglos, nach welchen Verfahren sie generiert wurden, d.h. mit oder ohne Verwendung von Phosgen.

Bevorzugt werden aliphatische Isocyanate und Senföle der vorstehend genannten Art eingesetzt.

Die als Ausgangsstoffe eingesetzten 3-Phospholene und deren Herstellungswege sind in der Literatur beschrieben: K. Dimroth in Comprehensive Heterocyclic Chemistry, Pergamon Press, Vol. 1, p. 514 ff, 1983 und darin zitierte Literatur.

Die neuen Substanzen der Klasse der 1,4,2-Diazaphospholidine sind kristalline Festkörper bzw. Flüssigkeiten und erweisen sich als wertvolle Produkte bzw. Zwischenprodukte für eine Reihe von Anwendungen, z.B. in der Katalyse. So sind sie interessante Katalysatoren für die anteilige Carbodiimid/Uretoniminbildung aus Isocyanaten sowie zur Katalyse der (anteiligen) Weiterreaktion der gebildeten Carbodiimidgruppen zu 6-Imino-1,3,5-triazin-2,4-dionen (Iminotriazindionen, Heterocyclen aus 2 mol Isocyanat und einem mol Carbodiimid).

Während die auf Isocyanaten bzw. Carbodiimiden basierenden Produkte der Formel (1) in der Regel farblos sind (sofern das zu Grunde liegende Isocyanat bzw. Carbodiimid kein Chromophor trägt) sind die erfindungsgemäßen Folgeprodukte der Isothiocyanate (Senföle) in der Regel gelb bis orange gefärbt.

Sie weisen die für Derivate des dreibindigen Phosphors typische Reaktivität auf und lassen sich z.B. durch (Luft)sauerstoff bzw. Schwefel oxidieren und durch Alkylierungsmittel am P-Atom alkylieren.

Doppelbindungen in den Edukten werden bei der erfindungsgemäßen Umsetzung toleriert.

Die erfindungsgemäße Reaktion kann, insbesondere in der aromatischen Reihe, auch ausgehend von Isocyanaten unter intermediärer Bildung der Carbodiimide - z.B. katalysiert durch kleine Mengen der den Verbindungen der Formel (2) entsprechenden P-Oxide (1-Organyl-2,5-dihydro-1H-phosphol-1-oxide, Phospholenoxide) die gleichzeitig im Reaktionsgemisch zugegen sein können - geführt werden, wobei in der Regel Gemische der Verbindungen (1) mit X = O und N-R2 anfallen.

Da sterisch anspruchsvolle aliphatische Isocyanate deutlich langsamer reagieren als linearaliphatische, sind durch geeignete Wahl der Reaktionsbedingungen auch gemischt substituierte Verbindungen wie z. B. der Formel (3) zugänglich. Der Alkylrest des reaktiveren Isocyanates findet sich dabei bevorzugt in der 4-Stellung des 1,4,2-Diazaphospholidin-3,5-dionringes wieder.

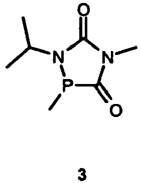

### Beispiele:

Alle Einsatzstoffe, soweit nicht anders vermerkt, sind Produkte der Firmen Bayer bzw. Lanxess.

Wenn nicht anders vermerkt, wurde unter striktem Luftausschluß unter einer trockenen Stickstoffatmosphäre gearbeitet.

Die eingesetzten 1-Organyl-2,5-dihydro-1H-phosphole (symm. Phospholene) wurden nach literaturbekannten Methoden gewonnen (vgl. K. Dimroth in Comprehensive Heterocyclic Chemistry, Pergamon Press, Vol. 1, p. 514 ff, 1983 und darin zit. Literatur).

¹H- sowie ³¹P-NMR spektroskopische Untersuchungen (letztere, wenn nicht anders angegeben, immer Protonen-entkoppelt) wurden an jeweils 10-15 mg Substanz in 0,7 ml trockenem, sauerstofffreiem CDCl₃ enthaltenden Lösungen auf den Geräten DPX 400, AVC 400 bzw. DRX 700 der Fa. Bruker, Karlsruhe, DE vorgenommen.

¹³C-NMR spektroskopische Untersuchungen wurden an ca. 30-50 %-igen Proben in trockenem, sauerstofffreiem CDCl₃ mit den o.g. Geräten vorgenommen. Als Referenz für die ppm-Skala wurden geringe Mengen Tetramethylsilan im entsprechenden Lösungsmittel (δ = 0 ppm) oder das Lösungsmittelsignal selbst (δ = 77,0 ppm) gewählt, positives Vorzeichen indiziert Verschiebung zu niedrigerem Feld relativ zum Standard.

Die charakteristischen NMR-Daten werden wie folgt angegeben:
<Kern>-NMR: <chem. Verschiebung [ppm]> (<Multiplizität: s = Singulett, d = Dublett, t = Triplett, q = Quartett, m = Multiplett>) <Kopplungskonstante [Hz]>.

Der koppelnde Kern ist, wenn nicht anders angegeben, Phosphor.

### Beispiel 1: 1,2,4-Trimethyl-1,4,2-diazaphospholidin-3,5-dion

Zu 1,4 g (24,5 mmol) auf 0°C gekühltem Methylisocyanat wurden im Verlaufe von 10 min 1,15 g (11,5 mmol) 1-Methyl-2,5-dihydro-1H-phosphol (symm. 1-Methylphospholen) getropft. Die Mischung wurde im langsam Zimmertemperatur annehmenden Kältebad weitere 4 h gerührt und anschließend analysiert. Neben den u. g. Signalen der Zielverbindung waren nur sehr intensitätsarme Signale von Verunreinigungen zu sehen. Das intensivste unter den Letzteren ist dem 1-Methyl-2,5-dihydro-1H-phosphol-1-oxid (symm. 1-Methylphospholenoxid) zuzuordnen (³¹P: 65,3 ppm (s)).

Die Vakuumdestillation des Reaktionsgemisches lieferte 1,75 g (95% d. Th) der Zielverbindung als farblose, leicht ölige Flüssigkeit; Kp 80°C/2 mbar.

| | |
|---|---|
| ³¹P-NMR: | 30,7 ppm (s) |
| ¹H-NMR: | 3,0 ppm (s); 2,9 ppm (d) 7,6 Hz; 1,4 ppm (d) 4,0 Hz, Integrationsverhältnis 1:1:1 |
| ¹³C-NMR: | 182,7 ppm (d) 2,9 Hz; 157,4 ppm (d) 5,8 Hz; 29,0 ppm (d) 16,5 Hz , 26,6 ppm (s), 15,5 ppm (d) 32,0 Hz |

Die Struktur der Zielverbindung wurde zusätzlich durch die Ergebnisse der hochauflösenden Massenspektrometrie: m/z = 160,04041 (ber.: 160,04017) sowie die Kristallstrukturanalyse des P-Sulfides (vgl. Bsp. 4) bestätigt.

### Beispiel 2: 1,2,4-Trimethyl-1,4,2-diazaphospholidin-3,5-dithion

320 mg (4,4 mmol) Methylisothiocyanat (Fa. ABCR) wurden zunächst im Vakuum bei 45°C Badtemperatur aufgeschmolzen (von gelösten Gasen befreit), mit Stickstoff beaufschlagt und anschließend nach Entfernen der externen Wärmequelle tropfenweise mit 200 mg (2 mmol) 1-Methyl-2,5-dihydro-1H-phosphol versetzt, wobei eine Farbvertiefung von gelb nach orange eintrat. Die orange Mischung erstarrte schnell, wurde 4 h bei 70°C gerührt und anschließend analysiert. Neben den u. g. Signalen der Zielverbindung waren nur sehr intensitätsarme Signale von Verunreinigungen zu sehen. Das intensivste hiervon war dem 1-Methyl-2,5-dihydro-1H-phosphol-1-sulfid (symm. 1-Methylphospholensulfid) zuzuordnen (³¹P: 55,3 ppm (s)).

Die Vakuumsublimation des Reaktionsgemisches bei 0,1 mbar/ 90°C Badtemperatur lieferte 340 mg (88% d. Th) der Zielverbindung als zunächst grüngelb fluoreszierenden Feststoff, der am nächsten Tag in eine zitronengelbe Kristallmasse übergegangen war, m.p.: 75°C.

| | |
|---|---|
| ³¹P-NMR: | 67,9 ppm (s) |
| ¹H-NMR: | 3,7 ppm (s); 3,3 ppm (d) 6,5 Hz; 1,6 ppm (d) 5,0 Hz, Integrationsverhältnis 1:1:1 |
| ¹³C-NMR: | 215,4 ppm (d) 21,5 Hz; 184,9 ppm (d) 5,6 Hz; 36,2 ppm (s); 34,8 ppm (d) 15,6 Hz; 20,0 ppm (d) 35,9 Hz |

### Beispiel 3: 1,4-Dibutyl-2-methyl-1,4,2-diazaphospholidin-3,5-dion

**a)** Zu 8,85 g (89,2 mmol) n-Butylisocyanat wurden bei Zimmertemperatur schnell 3,57 g (35,7 mmol) 1-Methyl-2,5-dihydro-1H-phosphol gegeben. Die farblose, flüssige Mischung erwärmte sich dabei nur geringfügig und verfärbte sich allmählich nach hellgelb. Nach 16 h Rühren bei Zimmertemperatur wurde mittel ¹H- und ³¹P-NMR analysiert. Im ³¹P-NMR-Spektrum waren 3 Singuletts bei 65,1 ppm (1-Methyl-2,5-dihydro-1H-phosphol-1-oxid), 24,1 ppm (Zielverbindung) sowie -41,0 ppm (1-Methyl-2,5-dihydro-1H-phosphol, Edukt) im Integrationsverhältnis 1: 17 : 35,7 zu sehen. Im ¹H-NMR Spektrum wurden neben den Signalen des nicht umgesetzten n-Butylisocyanates sowie der o.g. P-haltigen Edukte und (Neben-)Produkte die 3 charakteristischen Signalgruppen des 1,3-Butadiens detektiert (6,33 - 6,23 ppm (m); 5,20 - 5,11 ppm (m); 5,08 - 5,00 ppm (m); Integrationsverhältnis 1:1:1). Signale für Oligomere des n-Butylisocyanates waren nicht erkennbar.
Anschließend wurde weitere 70 h bei 50°C gerührt und danach destillativ aufgearbeitet. 7,4 g (85% d. Th) der Zielverbindung wurden als farblose, leicht ölige Flüssigkeit mit Kp 93°C/0,02 mbar erhalten.

| | |
|---|---|
| ³¹P-NMR: | 24,2 ppm (s) |
| ¹H-NMR: | ca. 3,4 ppm (m); ca. 3,3 ppm (m); ca. 1,5 ppm (m), 1,4 ppm (d) 4,5 Hz; ca. 1,2 ppm (m); 0,82 ppm (t) J_{HH} 7,05 Hz; 0,80 ppm (t) J_{HH} 7,55 Hz, Integrationsverhältnis 1:1:2:1,5:2:1,5:1,5 |
| ¹³C-NMR: | 183,0 ppm (d) 1,9 Hz; 157,2 ppm (d) 5,8 Hz; 42,9 ppm (d) 14,6 Hz, 40,2 ppm (s), 31,6 ppm (d) 3,1 Hz; 30,0 ppm (s); 19,8 ppm (s); 19,7 ppm (s); 16,9 ppm (d) 32,9 Hz; 13,5 ppm (s) |

Die Identität und Reinheit (ca. 95%ig) der Zielverbindung wurde zusätzlich mittels hochauflösender Massenspektrometrie: m/z = 244,13479 (ber.: 244,13407) untersucht. Die häufigsten Nebenkomponenten waren N,N'-Di-n-butylcarbodiimid (2,5 %) und 1,3,5-Tri-n-butyl-1,3,5-triazin-2,4,6-trion (Tri-n-butyl-isocyanurat, 1,4 %).
**b)** Zu 0,97 g (9,8 mmol) n-Butylisocyanat wurden bei Zimmertemperatur schnell 0,6 g (4,7 mmol) 1,3,4-Trimethyl-2,5-dihydro-1H-phosphol gegeben. Eine Exothermie war nicht zu beobachten. Die zunächst farblose, flüssige Mischung verfärbte sich allmählich nach hellgelb. Nach 16 h Rühren bei Zimmertemperatur wurde mittel ¹H- und ³¹P-NMR analysiert. Der Umsatz war deutlich weiter voran geschritten, als unter a), Signale von Nebenprodukten, außer dem P-Oxid (1,3,4-Trimethyl-2,5-dihydro-1H-phosphol-1-oxid) und seinen 2,3-Dihydro-Pendants (2 Isomere) bei 57,4 und 60,0/61,7 ppm (summarisch ca. 4 % lt. ³¹P-NMR), wurden nicht detektiert. Die destillative Aufarbeitung lieferte in 95%iger Ausbeute ein deutlich reineres Produkt (>99% lt. GC) als unter a) mit den unter a) aufgeführten physikalischen und spektroskopischen Daten.
**c)** Zu 1,74 g (17,5 mmol) n-Butylisocyanat wurden bei Zimmertemperatur schnell 0,8 g (7 mmol) 1,3-Dimethyl-2,5-dihydro-1H-phosphol gegeben. Die farblose, flüssige Mischung erwärmte sich nicht und verfärbte sich allmählich nach hellgelb. Nach 16 h Rühren bei Zimmertemperatur wurde mittel ¹H- und ³¹P-NMR analysiert. Der Umsatz lag zwischen dem unter a) und b) beobachteten. Signale von Nebenprodukten, außer den P-Oxiden (1,3-Dimethyl-2,5 (2,3)-dihydro-1H-phosphol-1-oxid, 66,4 (70,5) ppm, summarisch ca. 3,8 mol-% lt. ³¹P-NMR), wurden nicht detektiert. Die destillative Aufarbeitung lieferte in 94%iger Ausbeute ein Produkt mit zu b) vergleichbarer Reinheit mit den unter a) aufgeführten physikalischen und spektroskopischen Daten.

### Beispiel 4: 1,2,4-Trimethyl-1,4,2-diazaphospholidin-3,5-dion-2-sulfid

425 mg (2,6 mmol) 1,2,4-Trimethyl-1,4,2-diazaphospholidin-3,5-dion (vgl. Bsp. 1) wurden mit 157 mg (4,9 mmol) elementarem Schwefel vermischt und 24 h auf 70°C erhitzt. Anschließend wurde mit wenig Methylenchlorid digeriert, von ungelöstem Schwefel abfiltriert und durch Überschichten mit n-Hexan langsame Kristallisation induziert. Eine erste Kristallfraktion (50 mg, 9,8% d. Th, m.p. 95°C) enthielt den mittels Kristallstrukturanalyse untersuchten Einkristall. Die vorgeschlagene Struktur wurde bestätigt.

### Beispiel 5: 1,4-Dibutyl-2,2-dimethyl-3,5-dioxo-1,4,2-diazaphospholidin-2-iumiodid

820 mg (3,3 mmol) 1,4-Dibutyl-2-methyl-1,4,2-diazaphospholidin-3,5-dion (vgl. Bsp. 3) wurden mit 6,27 g (44 mmol) Methyliodid (Fa. Aldrich) vermischt und 24 h unter Rühren mit einem Magnetrührkem am Rückfluß erhitzt (Badtemperatur: auf 80°C). Die gelbe Mischung, deren Viskosität merklich angestiegen war, wurde ohne weitere Aufarbeitung NMR-spektroskopisch untersucht:

| | |
|---|---|
| ³¹P-NMR: | 39,2 ppm (95%), ein Nebenprodukt bei 13,8 ppm (vermutlich das P-Oxid des Eduktes, 5%) |
| ¹H-NMR: | (nur Signale des Salzes): ca. 4,0 ppm (m); ca. 3,7 ppm (m); 3,1 ppm (d) 14,6 Hz; ca. 1,7 ppm (m); ca. 1,6 ppm (m); ca. 1,4 ppm (m); ca. 1,3 ppm (m); 0,91 ppm (t) J_{HH} 7,6 Hz; 0,89 ppm (t) J_{HH} 7,55 Hz, Integrationsverhältnis 1:1:3:1:1:1:1:1,5:1,5 |

### Beispiel 6: 2,4-Dimethyl-1-(propan-2-yl)-1,4,2-diazaphospholidin-3,5-dion

Zu einer Mischung aus 0,41 g (7,2 mmol) Methylisocyanat und 6,13 g (72 mmol) 2-Propylisocyanat wurden bei Zimmertemperatur im Verlaufe von 1 h 0,7g (7 mmol) 1-Methyl-2,5-dihydro-1H-phosphol gegeben. Eine Exothermie war nicht zu beobachten. Die zunächst farblose, flüssige Mischung verfärbte sich allmählich nach hellgelb. Nach 48 stündigem Rühren bei Zimmertemperatur wurde mittels ¹H-, ³¹P-NMR und GC-MS analysiert. Es wurden neben nicht umgesetztem 1-Methyl-2,5-dihydro-1H-phosphol und geringen Mengen seines P-Oxides im wesentlichen 2 Singuletts im ³¹P-NMR Spektrum bei 30,8 ppm (1,2,4-Trimethyl-1,4,2-diazaphospholidin-3,5-dion , vgl. Bsp. 1) und 15,6 ppm (Verbindung 3) gefunden. Das Integrationsverhältnis belief sich auf 1: 2,2. Gaschromatografisch wurde dieses Verhältnis zu 1: 3,5 bestimmt. Die Anordnung der N-gebundenen exocyclischen Substituenten wurde mittels 2D-NMR abgesichert.

### Beispiel 7:

### 2-Methyl-1,4-di(prop-2-en-1-yl)-1,4,2-diazaphospholidin-3,5-dithion und 2-Methyl-1,4-di(prop-2-en-1-yl)-1,4,2-diazaphospholidin-3,5-dithion-2-sulfid

1,15 g (11,6 mmol) Allylisothiocyanat (Fa. ABCR) wurden bei Zimmertemperatur tropfenweise mit 56 mg (5,6 mmol) 1-Methyl-2,5-dihydro-1H-phosphol versetzt, wobei unter leichter Temperaturerhöhung eine deutliche Farbvertiefung von gelb nach rot-orange eintrat. Die rot-orange, flüssige Mischung wurde nach Stehen über Nacht mittels ³¹P-NMR analysiert: 64,3 ppm (s) und anschließend mit überschüssigem Schwefel, 220 mg (6,99 mmol), in das P-Sulfid überführt, ³¹P: 59,0 ppm (s), MS: Molekülion m/z 276 (70%), Basispeak: m/z 177, {M-AllyINCS}.

### Beispiel 8:

### 2-Methyl-1,4-diphenyl-1,4,2-diazaphospholidin-3,5-dion und N,N'-(2-Methyl-1,4-diphenyl-1,4,2-diazaphospholidin-3,5-diyliden)dianilin

Zu 2,8 g (23,5 mmol) Phenylisocyanat wurden im Verlaufe von 10 min 0,96 g (9,5 mmol) 1-Methyl-2,5-dihydro-1H-phosphol getropft. Die Mischung erwärmte sich dabei leicht, verfärbte sich nach gelb-orange und Feststoffabscheidung setzte ein. Nach 24 stündigem Rühren/Stehen bei Zimmertemperatur wurde in Methylenchlorid gelöst und mittels ³¹P-NMR und GC/MS analysiert.
³¹P-NMR: zwei Singuletts etwa gleicher Intensität bei 38,3 und 34,5 ppm.

GC/MS: neben Spuren von Diphenylcarbodiimid wurden ausschließlich die Zielverbindungen (ca. 1:1) sowie 1,3,5-Triphenyl-1,3,5-triazin-2,4,6-trion (Triphenylisocyanurat) und 1,3,5-Triphenyl-6-(phenylimino)-1,3,5-triazin-2,4-dion (aus 2 mol Phenylisocyanat und einem mol Diphenylcarbodiimid) detektiert.

### Beispiel 9: Anwendungsbeispiel zur Katalyse

Zu 20,9 g (0,12 mol) Hexamethylendiisocyanat wurden 1,7 g (7 mmol) der unter Beispiel 3b beschriebenen Verbindung gegeben und 24 h bei 100°C gerührt. Der Brechungsindex (n_{D}²⁰) der klaren, flüssigen Mischung stieg dabei von 1,4559 auf 1,4689 an. NMR-spektroskopisch wurde die Bildung von Uretoniminstrukturen nachgewiesen. Anschließend wurde durch Zugabe von 920 mg n-Butanol die Reaktion unterbrochen. Auch bei weiterem Rühren bei 100°C (4 h) war kein weiterer Anstieg des Brechungsindex der Mischung zu beobachten. Das NMR-Spektrum blieb unverändert.

## Patentansprüche

1. 1,4,2-Diazaphospholidine der Formel (1) 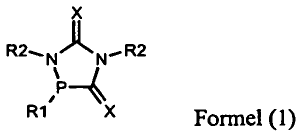 wobei
R1, R2 unabhängig voneinander gleiche oder verschiedene, ggf. durch Heteroatome (N, O, S, Halogen) substituierte bzw. unterbrochene, ggf. ein- oder mehrfach ungesättigte C₁-C₂₀-Alkyl-, -Alkenyl-, -Alkinyl- bzw. C₅-C₄₀-Arylgruppen sind und
X für gleiche oder verschiedene Substituenten aus der Gruppe O, S bzw. N-R2 steht.

2. 1,4,2-Diazaphospholidine gemäß Anspruch 1, **dadurch gekennzeichnet, dass** X Sauerstoff oder Schwefel ist.

3. Verfahren zur Herstellung von 1,4,2-Diazaphospholidinen der Formel (1) 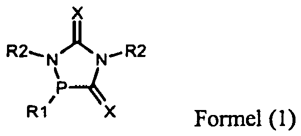 wobei
R1, R2 unabhängig voneinander gleiche oder verschiedene, ggf. durch Heteroatome (N, O, S, Halogen) substituierte bzw. unterbrochene, ggf. ein- oder mehrfach ungesättigte C₁-C₂₀-Alkyl-, -Alkenyl-, -Alkinyl- bzw. C₅-C₄₀-Arylgruppen sind und
X für gleiche oder verschiedene Substituenten aus der Gruppe O, S bzw. N-R2 steht,
in dem 3-Phospholene gemäß Formel (2) 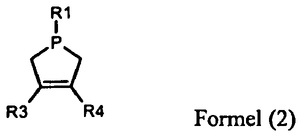 wobei
R1 die vorstehend genannte Bedeutung hat und
R3, R4 unabhängig voneinander gleiche oder verschiedene Substituenten aus der Gruppe H, Alkyl, Aryl sind
mit Verbindungen der Formel R2-NCX umgesetzt werden.

4. Verfahren gemäß Anspruch 3, **dadurch gekennzeichnet, dass** R3, R4 Wasserstoff oder eine Methylgruppe ist.

5. Verfahren gemäß Anspruch 3 oder 4, **dadurch gekennzeichnet, dass** die 3-Phospholene der Formel (2) im molaren Verhältnis von 1 : 2 bis 1 : 5 mit den Verbindungen R2-NCX umgesetzt werden.

6. Verwendung von 1,4,2-Diazaphospholidinen gemäß Anspruch 1 oder 2 als Katalysatoren.

7. Verwendung gemäß Anspruch 6, **dadurch gekennzeichnet, dass** es sich dabei um Katalysatoren für die zumindest anteilige Bildung von Carbodiimiden/Uretoniminen aus Isocyanaten oder für die zumindest anteilige Bildung von 6-Imino-1,3,5-triazin-2,4-dionen aus Carbodiimiden und Isocyanaten handelt.

## Claims

1. 1,4,2-Diazaphospholidines of the formula (1) 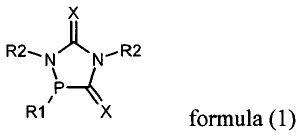 where
R1, R2 are each independently identical or different saturated or mono- or polyunsaturated C₁-C₂₀-alkyl, -alkenyl, -alkynyl or C₅-C₄₀-aryl groups optionally substituted or interrupted by heteroatoms (N, O, S, halogen), and
X represents identical or different substituents from the group of O, S and N-R2.

2. 1,4,2-Diazaphospholidines according to Claim 1, **characterized in that** X is oxygen or sulphur.

3. Process for preparing 1,4,2-diazaphospholidines of the formula (1) 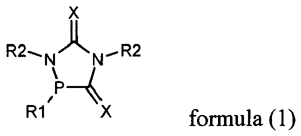 where
R1, R2 are each independently identical or different saturated or mono- or polyunsaturated C₁-C₂₀-alkyl, -alkenyl, -alkynyl or C₅-C₄₀-aryl groups optionally substituted or interrupted by heteroatoms (N, O, S, halogen), and
X represents identical or different substituents from the group of O, S and N-R2,
in which 3-phospholenes of the formula (2) 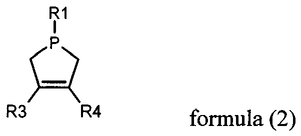 where
R1 is as defined above and
R3, R4 are each independently identical or different substituents from the group ofH, alkyl, aryl,
are reacted with compounds of the formula R2-NCX.

4. Process according to Claim 3, **characterized in that** R3, R4 are each hydrogen or a methyl group.

5. Process according to Claim 3 or 4, **characterized in that** the 3-phospholenes of the formula (2) are reacted with the compounds R2-NCX in a molar ratio of 1 : 2 to 1 : 5.

6. Use of 1,4,2-diazaphospholidines according to Claim 1 or 2 as catalysts.

7. Use according to Claim 6, **characterized in that** the catalysts are those for the at least partial formation of carbodiimides/uretonimines from isocyanates, or for the at least partial formation of 6-imino-1,3,5-triazine-2,4-diones from carbodiimides and isocyanates.

## Revendications

1. 1,4,2-diazaphospholidines de formule (1) 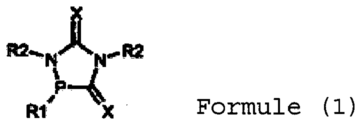 dans laquelle
R1, R2 représentent indépendamment l'un de l'autre des groupes alkyle en C₁-C₂₀, alcényle en C₁-C₂₀, alcynyle en C₁-C₂₀ ou aryle en C₅-C₄₀ identiques ou différents, éventuellement une ou plusieurs fois insaturés, éventuellement substitués ou interrompus par des hétéroatomes (N, O, S, halogène) et
X représente des substituants identiques ou différents, choisis dans l'ensemble constitué par O, S et N-R2.

2. 1,4,2-diazaphospholidines selon la revendication 1, **caractérisées en ce que** X est un atome d'oxygène ou de soufre.

3. Procédé pour la préparation de 1,4,2-diazaphospholidines de formule (1) 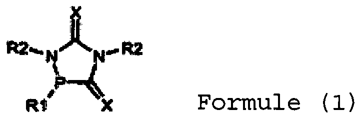 dans laquelle
R1, R2 représentent indépendamment l'un de l'autre des groupes alkyle en C₁-C₂₀, alcényle en C₁-C₂₀, alcynyle en C₁-C₂₀ ou aryle en C₅-C₄₀ identiques ou différents, éventuellement une ou plusieurs fois insaturés, éventuellement substitués ou interrompus par des hétéroatomes (N, O, S, halogène) et
X représente des substituants identiques ou différents, choisis dans l'ensemble constitué par O, S et N-R2,
dans lequel on fait réagir des 3-phospholènes selon la formule (2) 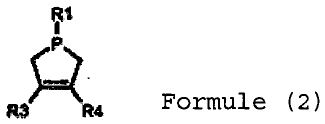 dans laquelle
R1 a la signification donnée précédemment et
R3, R4 représentent indépendamment l'un de l'autre des substituants identiques ou différents, choisis dans l'ensemble constitué par H, alkyle, aryle
avec des composés de formule R2-NCX.

4. Procédé selon la revendication 3, **caractérisé en ce que** R3, R4 représentent un atome d'hydrogène ou un groupe méthyle.

5. Procédé selon la revendication 3 ou 4, **caractérisé en ce qu'**on fait réagir les 3-phospholènes de formule (2) en un rapport molaire de 1:2 à 1:5 avec les composés R2-NCX.

6. Utilisation des 1,4,2-diazaphospholidines selon la revendication 1 ou 2 en tant que catalyseurs.

7. Utilisation selon la revendication 6, **caractérisée en ce qu'**en ce cas il s'agit de catalyseurs pour la formation au moins en une certaine proportion de carbodiimides/urétonimines à partir d'isocyanates ou pour la formation au moins en une certaine proportion de 6-imino-1,3,5-triazine-2,4-diones à partir de carbodiimides et d'isocyanates.
